(19) Europäisches Patentamt
European Patent Office
Office européen des brevets

(11) Veröffentlichungsnummer: **0 091 023**
**B1**

(12) # EUROPÄISCHE PATENTSCHRIFT

(45) Veröffentlichungstag der Patentschrift: **21.11.84**

(21) Anmeldenummer: **83102920.2**

(22) Anmeldetag: **24.03.83**

(51) Int. Cl.³: **C 07 C 145/02**, C 07 C 145/00, C 07 C 149/32, C 07 C 149/34, C 07 D 295/22, C 07 D 295/18, A 01 N 41/00, A 01 N 33/22, A 01 N 43/54, A 01 N 43/46

(54) **Herbizide Diphenylether, Verfahren zu ihrer Herstellung und ihre Verwendung zur Bekämpfung unerwünschten Pflanzenwuchses.**

(30) Priorität: **01.04.82 DE 3212165**

(43) Veröffentlichungstag der Anmeldung: **12.10.83 Patentblatt 83/41**

(45) Bekanntmachung des Hinweises auf die Patenterteilung: **21.11.84 Patentblatt 84/47**

(84) Benannte Vertragsstaaten: **BE DE FR GB IT NL**

(56) Entgegenhaltungen: **EP-A-0 022 317**

(73) Patentinhaber: **BASF Aktiengesellschaft, Carl-Bosch-Strasse 38, D-6700 Ludwigshafen (DE)**

(72) Erfinder: **Parg, Adolf, Dr., Paray-le-Monial-Strasse 8, D-6702 Bad Duerkheim (DE)**
Erfinder: **Ziegler, Hans, Dr., Am Speyerweg 48, D-6704 Mutterstadt (DE)**
Erfinder: **Wuerzer, Bruno, Dr. Dipl.-Landwirt, Ruedigerstrasse 13, D-6701 Otterstadt (DE)**

**EP 0 091 023 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Diphenylether, Verfahren zu ihrer Herstellung, Herbizide, die diese Verbindungen als Wirkstoffe enthalten sowie ihre Verwendung zur Bekämpfung unerwünschten Pflanzenwuchses.

Aus der Literatur sind Wirkstoffe aus der Klasse der Diphenylether bekannt, die in ortho-Stellung zur Nitrogruppe einen Thiorest besitzen (JP-OS 77/21 320, DE-OS 2 833 021).

Es wurde gefunden, daß Diphenylether der Formel

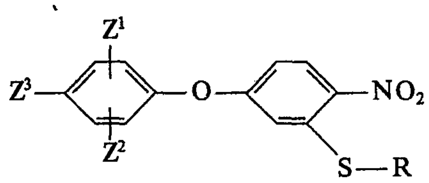

(I)

in der

$Z^1$ und $Z^2$ jeweils unabhängig voneinander Wasserstoff, Halogen, Nitro, Cyano, Carboxy, $C_1-C_4$-Alkyl, $C_1-C_4$-Halogenalkyl oder $C_1-C_4$-Alkoxy,

$Z^3$ Halogen, Nitro, Cyano, $C_1-C_4$-Alkyl, $C_1-C_4$-Halogenalkyl, $C_1-C_4$-Alkoxy, $C_1-C_4$-Halogenalkoxy, $C_1-C_4$-Alkylmercapto, $C_1-C_4$-Halogenalkylmercapto, $C_1-C_4$-Alkylsulfinyl, $C_1-C_4$-Halogenalkylsulfinyl, $C_1-C_4$-Alkylsulfonyl oder $C_1-C_4$-Halogenalkylsulfonyl und

R Wasserstoff, Halogen oder den Rest $-X-R^1$, wobei X für Sauerstoff und Schwefel und $R^1$ für Wasserstoff, $C_1-C_{20}$-Alkyl, $C_1-C_{20}$-Alkoxyalkyl, $C_1-C_{20}$-Alkylthioalkyl, $C_2-C_{20}$-Alkylaminoalkyl, $C_3-C_{20}$-Dialkylaminoalkyl, $C_1-C_{20}$-Halogenalkyl, einen gegebenenfalls durch Halogen substituierten araliphatischen Rest mit 7 bis 20 Kohlenstoffatomen, $C_2-C_8$-Alkenyl, $C_2-C_8$-Halogenalkenyl oder $C_2-C_8$-Alkinyl mit bis zu 8 Kohlenstoffatomen, Carboxyalkyl, Alkoxycarbonylalkyl oder Carbamoylalkyl mit bis zu 6 Kohlenstoffatomen, Cycloalkyl mit 3 bis 20 Kohlenstoffatomen oder gegebenenfalls substituiertes Phenyl stehen,
oder den Rest $-NR^2R^3$, wobei $R^2$ und $R^3$ gleich oder verschieden sind und die für $R^1$ genannten Bedeutungen haben oder wobei für den Fall, daß $R^3$ für Wasserstoff oder $C_1-C_4$-Alkyl steht, $R^2$ für Hydroxy, $C_1-C_{20}$-Alkoxy, durch $C_1-C_6$-Alkoxy, $C_1-C_6$-Alkylthio, $C_1-C_6$-Alkylamino, $C_2-C_6$-Dialkylamino, durch Halogen, Carboxyalkyl, Alkoxycarbonyl oder Phenyl substituiertes $C_1-C_{20}$-Alkoxy, gegebenenfalls substituiertes Phenoxy, Amino, durch $C_1-C_{20}$-Alkyl oder Phenyl substituiertes Amino steht oder wobei $R^2$ und $R^3$ zusammen für eine gegebenenfalls durch $C_1-C_4$-Alkyl ein- oder mehrfach substituierte Alkylenkette mit 4 oder 5 Kohlenstoffatomen stehen, die durch Sauerstoff oder Stickstoff unterbrochen sein kann, bedeuten,

je nach Aufbereitung und Dosierung total oder selektiv herbizid wirksam sind.

$Z^1$ und $Z^2$ in Formel I bedeuten jeweils unabhängig voneinander Wasserstoff, Halogen, Nitro, Cyano, Carboxy, $C_1-C_4$-Alkyl, $C_1-C_4$-Halogenalkyl oder $C_1-C_4$-Alkoxy, beispielsweise Fluor, Chlor, Brom, Jod, Methyl, Ethyl, n-Propyl, i-Propyl, n-Butyl, tert.-Butyl, Trifluormethyl, Difluormethyl, Fluormethyl, Trichlormethyl, Dichlormethyl, Chlormethyl, Difluorchlormethyl, 1-Chlorethyl, 2-Chlorethyl, 1-Fluorethyl, 2-Fluorethyl, 2,2,2-Trichlorethyl, 2,2,2-Trifluorethyl, 1,1,2,2-Tetrafluorethyl, 1,1,2-Trifluor-2-chlorethyl, 1,1,2,2,2-Pentafluorethyl, Methoxy, Ethoxy, n-Propyloxy, i-Propyloxy oder tert.-Butyloxy;

$Z^3$ bedeutet Halogen, Nitro, Cyano, $C_1-C_4$-Alkyl, $C_1-C_4$-Halogenalkyl, $C_1-C_4$-Alkoxy, $C_1-C_4$-Halogenalkoxy, $C_1-C_4$-Alkylmercapto, $C_1-C_4$-Halogenalkylmercapto, $C_1-C_4$-Alkylsulfinyl, $C_1-C_4$-Halogenalkylsulfinyl, $C_1-C_4$-Alkylsulfonyl oder $C_1-C_4$-Halogenalkylsulfonyl, beispielsweise $Z^3$ Fluor, Chlor, Brom, Jod, Methyl, Ethyl, n-Propyl, i-Propyl, n-Butyl, tert.-Butyl, Trifluormethyl, Difluormethyl, Fluormethyl, Trichlormethyl, Dichlormethyl, Chlormethyl, Difluorchlormethyl, 1-Chlorethyl, 2-Chlorethyl, 1-Fluorethyl, 2-Fluorethyl, 2,2,2-Trichlorethyl, 2,2,2-Trifluorethyl, 1,1,2,2-Tetrafluorethyl, 1,1,2-Trifluor-2-chlorethyl, 1,1,2,2,2-Pentafluorethyl, Methoxy, Ethoxy, n-Propyloxy, i-Propyloxy, tert.-Butyloxy, Trichlormethoxy, Trifluormethoxy, 1-Chlorethoxy, 2-Chlorethoxy, 1-Fluorethoxy, 2-Fluorethoxy, 2,2,2-Trichlorethoxy, 2,2,2-Trifluorethoxy, 1,1,2,2-Tetrafluorethoxy, 1,1,2,2,2-Pentafluorethoxy, Methylmercapto, Ethylmercapto, Trichlormethylmercapto, Trifluormethylmercapto, Methylsulfinyl, Ethylsulfinyl, Methylsulfonyl, Ethylsulfonyl oder Trifluormethylsulfonyl.

R in Formel I steht für Wasserstoff, Halogen, wie Fluor, Chlor, Brom, einen Rest $-XR^1$ oder einen Rest $-NR^2R^3$.

Im Rest $-XR^1$ steht $R^1$ für Wasserstoff, für Alkyl mit 1 bis 20, vorzugsweise 1 bis 4 Kohlenstoffatomen, beispielsweise Methyl, Ethyl, n-Propyl, 2-Propyl, n-Butyl, tert.-Butyl, isomere Pentylreste, isomere Hexylreste, isomere Heptylreste, für Alkoxyalkyl mit 2 bis 20, insbesondere 2 bis 6 Kohlenstoffatomen, wie Methoxymethyl, Ethoxymethyl, Methoxyethyl, Methoxypropyl, Methoxybutyl, Ethoxybutyl, für Alkylthioalkyl mit 2 bis 20, insbesondere 2 bis 6 Kohlenstoffatomen, wie Methylthiomethyl, Ethylthiomethyl, Methylthioethyl, Methylthiopropyl, Methylthiobutyl, Ethylthiopropyl, für Alkylaminoalkyl mit 2 bis 20, insbesondere 2 bis 6 Kohlenstoffatomen, wie Methylaminomethyl, Methylaminomethyl, Ethylaminomethyl, Ethylaminomethyl, Methylaminopropyl, Methylaminobutyl, für Dialkylaminoalkyl

mit 3 bis 20, insbesondere 3 bis 8 Kohlenstoffatomen, wie Dimethylaminomethyl, Dimethylaminoethyl, Diethylaminoethyl, Diethylaminopropyl, Diethylaminobutyl, für Halogenalkyl mit 1 bis 20, insbesondere 1 bis 4 Kohlenstoffatomen, wie Chlormethyl, Trichlormethyl, 2-Chlorethyl, 2-Fluorethyl, 3-Chlorpropyl, 4-Chlorbutyl, für einen gegebenenfalls durch Halogen substituierten araliphatischen Rest mit 7 bis 20 Kohlenstoffatomen, insbesondere 7 bis 9 Kohlenstoffatomen, wie Benzyl, 4-Chlorbenzyl, 2,4-Dichlorbenzyl, Phenethyl, Phenpropyl, für Alkenyl, Halogenalkenyl oder Alkinyl mit jeweils 2 bis 8, insbesondere 2 bis 4 Kohlenstoffatomen, wie Ethenyl, Propen-1-yl, Propben-2-yl, Chlorethenyl, Ethinyl, Propargyl, Butinylreste, für einen Carboxyalkylrest der Formel $-(CHR^4)_n-COOH$, in der $R^4$ Wasserstoff, Methyl, Ethyl oder Propyl und n die Zahlen 1, 2 oder 3 bedeuten, für einen Alkoxycarbonylalkylrest der Formel $-(CHR^4)_nCOOR^5$, in der $R^4$ und n die obengenannten Bedeutungen haben und $R^5$ für Alkyl mit 1 bis 20, insbesondere 1 bis 4 Kohlenstoffatomen, wie Methyl, Ethyl, Propyl, Butyl, steht, für einen Carbamoylalkylrest der Formel $-(CHR^4)_n-CO-NR^6R^7$, in der $R^4$ und n die obengeannten Bedeutungen haben und $R^6$ und $R^7$ gleich oder verschieden sind und Wasserstoff, $C_1-C_4$-Alkyl, wie Methyl oder Ethyl, einen gegebenenfalls durch Halogen, $C_1-C_4$-Alkyl, $C_1-C_4$-Halogenalkyl, wie Trifluormethyl, oder Cyano substituierten Phenylring bedeuten oder wobei $R^6$ und $R^7$ zusammen eine Alkylenkette mit 4 bis 6 Kohlenstoffatomen bilden, für einen Cycloalkylrest mit 3 bis 20, insbesondere 3 bis 6 Kohlenstoffatomen, wie Cyclopropyl, Cyclobutyl, Cyclopentyl, Cyclohexyl, oder für einen gegebenenfalls durch Halogen, $C_1-C_4$-Alkyl, wie Methyl, Ethyl, $C_1-C_4$-Halogenalkyl, wie Trifluormethyl, Cyano oder einen Alkoxycarbonylalkoxyrest der Formel $-O-(CHR^4)_n-COOR^5$, in der $R^4$, $R^5$ und n die obengenannten Bedeutungen haben, wie 1-n-Butoxycarbonyl-ethoxy, substituiertes Phenyl.

$R^2$ und $R^3$ im Rest $-NR^2R^3$ sind gleich oder verschieden und haben die für $R^1$ genannten Bedeutungen. Für den Fall, daß $R^3$ für Wasserstoff oder $C_1-C_4$-Alkyl steht, kann $R^2$ auch für Hydroxy, für Alkoxy mit 1 bis 20, vorzugsweise 1 bis 4 Kohlenstoffatomen, wie Methoxy, Ethoxy, Propoxy, Butoxy, für durch $C_1-C_8$-Alkoxy, $C_1-C_8$-Alkylthio, $C_1-C_8$-Alkylamino, $C_2-C_8$-Dialkylamino, Halogen, Carboxyalkyl, Alkoxycarbonyl oder Phenyl substituiertes Alkoxy mit 1 bis 20 Kohlenstoffatomen, wie Methoxymethoxy, Methoxyethoxy, Methylthiomethoxy, Methylthioethoxy, Methylaminoethoxy, Dimethylaminoethoxy, Chlorethoxy, Fluorethoxy, Carbonylmethoxy, Methoxycarbonylmethoxy, 2-Methoxycarbonylethoxy, Benzyloxy, Phenethyloxy, für gegebenenfalls durch Halogen oder $C_1-C_4$-Alkyl substituiertes Phenoxy, wie Phenoxy, 4-Chlorphenoxy, 2,4-Dichlorphenoxy, 4-Methylphenoxy, für Amino, für ein durch Alkyl mit 1 bis 20, vorzugsweise 1 bis 8 Kohlenstoffatomen, oder durch gegebenenfalls durch Halogen, Cyano, Nitro, $C_1-C_4$-Alkyl, $C_1-C_4$-Haogenalkyl substituiertes Phenyl substituiertes Amino, wie Methylamino, Ethylamino, Propylamino, Butylamino, Pentylamino, Phenylamino, 4-Chlorphenylamino, 2,4-Dichlorphenylamino, 4-Cyanphenylamino, 3-Trifluormethylphenylamino, 4-Methylphenylamino, 4-Nitrophenylamino stehen. $R^2$ und $R^3$ können auch zusammen eine gegebenenfalls durch $C_1-C_4$-Alkyl ein- oder mehrfach substituierte Alkylenkette mit 4 oder 5 Kohlenstoffatomen, die durch Sauerstoff oder Stickstoff unterbrochen sein kann, bilden. Mit dem Stickstoffatom, dessen Substituenten sie sind, bilden sie dann beispielsweise folgende Reste: Azetidino, Pyrrolidino, Piperidino, Morpholino, 2,6-Dimethylmorpholino, Piperazino, 2,6-Dimethylpiperazino, N-Methylpiperazino, N-Ethylpiperazino. Die Substituenten $Z^1$, $Z^2$ und $Z^3$ stehen vorzugsweise in 2,4,6-Stellung am Phenylring.

Bevorzugte Diphenylether sind Verbindungen der Formel I, in der $Z^1$, $Z^2$ und $Z^3$ jeweils unabhängig voneinander Wasserstoff, Halogen, $C_1-C_4$-Halogenalkyl oder $C_1-C_4$-Halogenalkylmercapto und R den Rest $-X-R^1$, wobei X für Sauerstoff oder Schwefel und $R^1$ für $C_1-C_8$-Alkyl, $C_2-C_6$-Alkoxyalkyl oder $C_2-C_6$-Alkylthioalkyl, $C_2-C_6$-Carboxyalkyl oder $C_3-C_6$-Alkoxycarbonylalkyl stehen, bedeuten. Bevorzugt sind auch Verbindungen, bei denen $Z^1$, $Z^2$ und $Z^3$ die zuletzt genannten Bedeutungen haben und R den Rest $-NR^2R^3$ bedeutet, wobei $R^2$ und $R^3$ gleich und verschieden sind und für Wasserstoff, $C_1-C_{10}$-Alkyl, $C_2-C_6$-Alkoxyalkyl oder $C_2-C_6$-Alkylthioalkyl oder gegebenenfalls durch Halogen substituiertes Phenyl oder wobei $R^3$ für Wasserstoff oder $C_1-C_4$-Alkyl und $R^2$ für $C_1-C_4$-Alkoxy, Amino oder durch $C_1-C_8$-Alkyl substituiertes Amino stehen. Insbesondere bevorzugt sind Diphenylether der Formel I, in der $Z^1$ Wasserstoff, $Z^2$ und $Z^3$ Halogen oder $C_1-C_4$-Halogenalkyl und R den Rest $-X-R^1$, wobei X für Sauerstoff und $R^1$ für $C_1-C_4$-Alkyl stehen, bedeuten.

Diphenylether der Formel I, in der R Halogen bedeutet, erhält man durch Umsetzung von Ethern der Formel

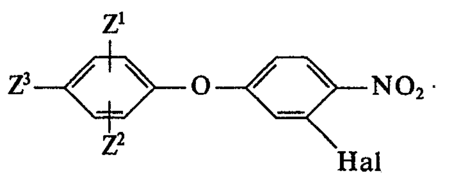
(II)

in der $Z^1$, $Z^2$ und $Z^3$ die obengenannten Bedeutungen haben und Hal für Halogen steht, mit der mindestens stöchiometrischen Menge Natriumdisulfid in Gegenwart eines organischen Lösungsmittels bei Temperaturen im Bereich von 0 bis 120°C, insbesondere von 20 bis 80°C, zu dem Disulfid der Formel

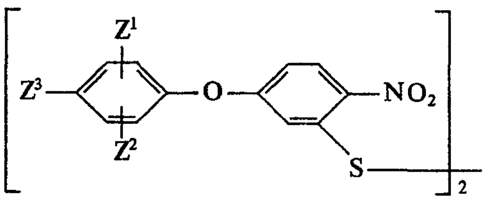

(III)

in der $Z^1$, $Z^2$ und $Z^3$ die obengenannten Bedeutungen haben und anschließende Umsetzung dieses Disulfids mit mindestens der stöchiometrischen Menge an elementarem Halogen in Gegenwart eines inerten organischen Lösungsmittels bei einer Temperatur im Bereich von −20 bis +120°C, insbesondere 0 bis 100°C. Beide Umsetzungen können diskontinuierlich oder kontinuierlich, drucklos oder unter Druck durchgeführt werden.

Diphenylether der Formel I, in der R Wasserstoff, den Rest $-XR^1$ oder den Rest $-NR^2R^3$ bedeutet, erhält man durch Umsetzung von Sulfenylhalogeniden der Formel

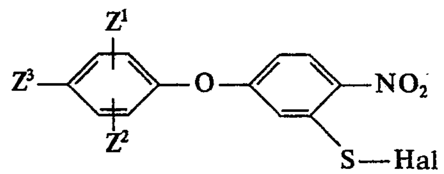

(IV)

in der $Z^1$, $Z^2$ und $Z^3$ die obengenannten Bedeutungen haben und Hal für Halogen steht, mit der mindestens äquimolaren Menge einer Verbindung der Formel

$$R-H \qquad (V)$$

in der R die obengenannten Bedeutungen hat, in einem inerten organischen Lösungsmittel und gegebenenfalls in Gegenwart eines Säureacceptors bei Temperaturen von −20 bis +150°C, insbesondere 0 bis 120°C. Die Umsetzung kann diskontinuierlich oder kontinuierlich, drucklos oder unter Druck durchgeführt werden.

Das Verfahren zur Herstellung der Disulfide der Formel III läßt sich durchfolgendes Formelschema wiedergeben:

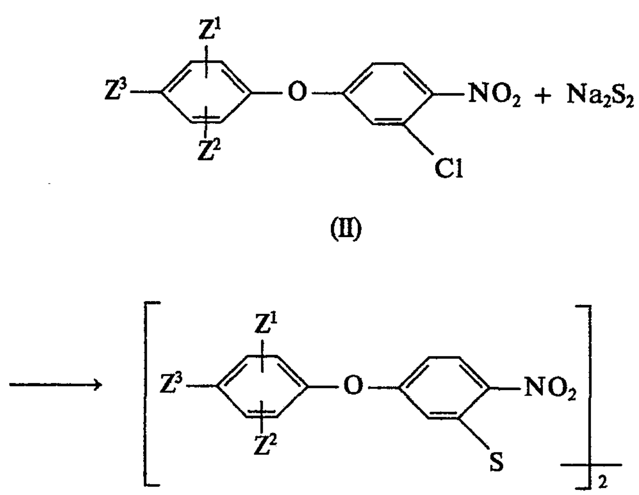

(II)

(III)

Die Ausgangsstoffe werden in ungefähr stöchiometrischem Verhältnis eingesetzt, vorzugsweise jedoch in einem Überschuß von bis zu 10% an Natriumdisulfid, bezogen auf II. Das Verfahren wird zweckmäßigerweise so durchgeführt, daß man eine Suspension von Natriumdisulfid in einem organischen Lösungsmittel mit der äquimolaren Menge an Halogenverbindung II versetzt. Zur Beendigung der Umsetzung rührt man 0,5 bis 48 Stunden, vorzugsweise 2 bis 12 Stunden, bei 0 bis 80°C nach. Das Reaktionsgemisch wird abgesaugt oder eingeengt. Die gewünschten Endstoffe können durch Umfällen, Umkristallisieren oder durch Verrühren mit Wasser isoliert werden.

Das Verfahren zur Herstellung der Sulfenylhalogenide der Formel IV läßt sich durch folgendes Formelschema wiedergeben:

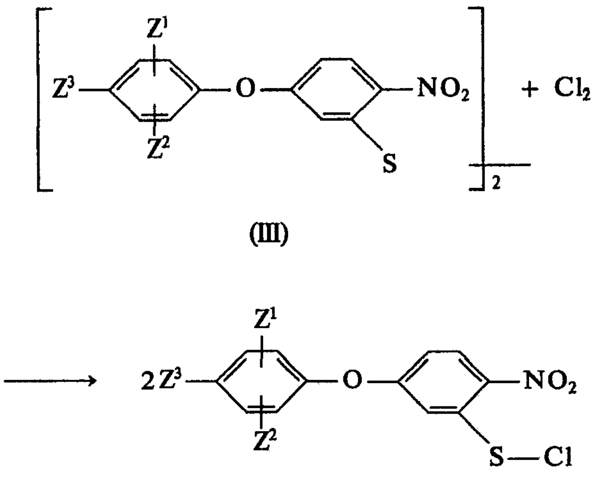

(III)

(IV)

Die Ausgangsstoffe werden in ungefähr stöchiometrischem Verhältnis eingesetzt, vorzugsweise jedoch in einem Überschuß von bis zu 30% an Halogen, bezogen auf III. Das Verfahren wird zweckmäßigerweise so durchgeführt, daß man eine Suspension oder Lösung des Disulfids in einem inerten organischen Lösungsmittel mit der mindestens äquimolaren Menge an Halogen versetzt. Zur Beendigung der Reaktion rührt man 0,5 bis 48 Stunden, vorzugsweise 2 bis 12 Stunden, bei 0 bis 100° C nach. Das Reaktionsgemisch wird eingeengt und die gewünschten Endstoffe durch Umfällen, Umkristallisieren oder Chromatographieren isoliert.

Das Verfahren zur Herstellung der Diphenylether der Formel I, in der R Wasserstoff, den Rest $-XR^1$ oder den Rest $-NR^2R^3$ bedeutet, läßt sich beispielhaft durch folgendes Formelschema wiedergeben:

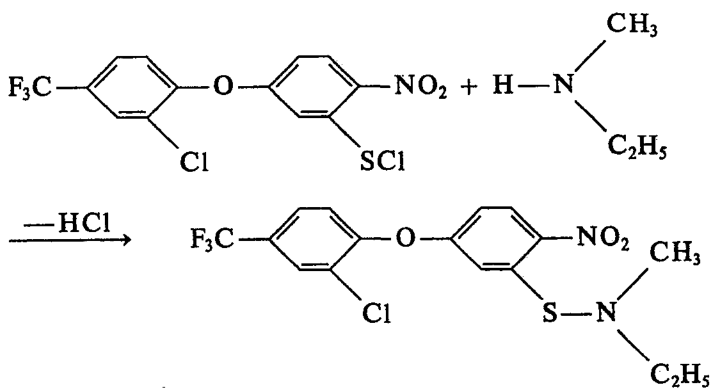


Die Ausgangsstoffe werden in ungefähr stöchiometrischem Verhältnis eingesetzt, d. h. in einem Unter- bzw. Überschuß von bis zu 10% an Ausgangsstoff V, bezogen auf IV. Gegebenenfalls kann ein Säureacceptor zur Vervollständigung der Reaktion zugesetzt werden, wobei diese Funktion auch für den Fall, daß der Ausgangsstoff V ein Amin darstellt, von diesem selbst übernommen werden kann. Ferner kann der bei der Umsetzung entstehende Halogenwasserstoff auch durch Einleiten eines inerten Gases, wie Stickstoff, ausgetrieben werden. Zweckmäßigerweise wird das Verfahren so durchgeführt, daß man eine Lösung des Sulfenylhalogenids IV bei −20 bis +150° C, vorzugsweise von 0 bis +120° C, in einem inerten organischen Lösungsmittel, gegebenenfalls gleichzeitig mit der äquimolaren Menge eines Säureacceptors, zu einer Lösung des Ausgangsstoffes V in einem inerten organischen Lösungsmittel zulaufen läßt.

Zur Beendigung der Umsetzung rührt man 0,5 bis 48 Stunden, vorzugsweise 2 bis 12 Stunden bei 0 bis 60° C nach. Das Reaktionsgemisch wird eingeengt. Die gewünschten Endstoffe können durch Umfällen, Umkristallisieren oder durch Verrühren mit Wasser isoliert werden; gegebenenfalls können sie durch Chromatographie gereinigt werden.

Für beide Verfahren verwendet man unter den jeweiligen Reaktionsbedingungen inerte organische Lösungsmittel. Als Lösungsmittel kommen beispielsweise Halogenkohlenwasserstoffe, insbesondere Chlorkohlenwasserstoffe, z. B. Tetrachlorethylen, 1,1,2,2- oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzol, Jodbenzol, o-, p- und m-Dichlorbenzol, o-, p-, m-Dibrombenzol, o-, m-, p-Chlortoluol, 1,2,4-Trichlorbenzol; Ether, z. B. Ethylpropylether, Methyl-tert.-butylether, n-Butylethylether, Di-n-butylether, Diisobutylether, Diisoamylether, Diisopropylether, Anisol, Phenetol, Cyclohexylmethylether, Diethylether, Ethylenglykoldimethylether, Tetrahydrofuran, Dioxan, Thioanisol, $\beta,\beta'$-Dichlordiethylether; Nitrokohlenwasserstoffe wie Nitromethan, Nitroethan, Nitrobenzol, o-, m-, p-Chlornitrobenzol, o-Nitrotoluol; Nitrile wie Acetonitril, Butyronitril, Isobutyronitril, Benzonitril, m-Chlorbenzonitril; aliphatische oder cycloaliphatische Kohlenwasserstoffe, z. B. Heptan, Pinan, Nonan, o-, m-, p-Cyamol, Benzinfraktionen innerhalb eines Siedepunktintervalls von 70 bis 190°C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan; Ester, z. B. Ethylacetat, Acetessigester, Isobutylacetat; Amide, z. B. Formamid, Methylformamid, Dimethylformamid; Ketone, z. B. Aceton, Methylethylketon; und entsprechende Gemische in Betracht. Zweckmäßig verwendet man das Lösungsmittel in einer Menge von 100 bis 2000 Gew.-%, vorzugsweise von 200 bis 700 Gew.-%, bezogen auf die Ausgangsstoffe.

Als Säureacceptoren können alle üblichen Säurebindemittel verwendet werden. Hierzu gehören vorzugsweise tertiäre Amine, Erdalkaliverbindungen, Ammoniumverbindungen und Alkaliverbindungen sowie entsprechende Gemische. Es können aber auch Zinkverbindungen verwendet werden. Es kommen z. B. basische Verbindungen in Frage: Kaliumhydroxid, Natriumhydroxid, Kaliumcarbonat, Natriumcarbonat, Lithiumhydroxid, Lithiumcarbonat, Natriumbicarbonat, Kaliumbicarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumbicarbonat, Magnesiumacetat, Zinkhydroxid, Zinkoxid, Zinkcarbonat, Zinkbicarbonat, Zinkacetat, Natriumformiat, Natriumacetat, Trimethylamin, Triethylamin, Tripropylamin, Triisopropylamin, Tributylamin, Triisobutylamin, Tri-sec.-butylamin, Tri-tert.-butylamin, Tribenzylamin, Tricyclohexylamin, Triamylamin, Diisopropylethylamin, Trihexylamin, N,N-Dimethylanilin, N,N-Diethylanilin, N,N-Dipropylanilin, N,N-Dimethyltoluoidin, N,N-Diethyltoluidin, N,N-Dipropyltoluidin, N,N-Dimethyl-p-aminopyrridin, N,N-Diethyl-p-aminopyridin, N,N-Dipropyl-p-aminopyridin, N-Methylpyrrolidon, N-Ethylpyrrolidon, N-Methylpiperidin, N-Ethylpiperidin, N-Methylpyrrolidin, N-Ethylpyrrolidin, N-Methylimidazol, N-Ethylimidazol, N-Methylpyrrol, n-Ethylpyrrol, N-Methylmorpholin, N-Ethylmorpholin, N-Methylhexamethylenimin, N-Ethylhexamethylenimin, Pyridin, Chinolin, $\alpha$-Picolin, $\beta$-Picolin, $\gamma$-Picolin, Isochinolin, Pyrimidin, Acridin, N,N,N',N'-Tetramethylethylendiamin, N,N,N',N'-Tetraethylethylendiamin, Chinoxalin, Chinazolin, N-Propyldiisopropylamin, N,N'-Dimethylcyclohexylamin, 2,6-Lutidin, 2,4-Lutidin, Trifurylamin, Triethylendiamin.

Die Ausgangsverbindungen werden nach literaturbekannten Methoden hergestellt. So erhält man die Ether der Formel II nach der in der DE-OS 2 311 638 beschriebenen Verfahrensweise. Die Verbindungen der Formel V sind allgemein zugänglich oder können ebenfalls nach literaturbekannten Verfahren hergestellt werden.

Die folgenden Beispiele erläutern die Herstellung der Verbindungen der Formel I nach dem angegebenen Verfahren. Gewichtsteile verhalten sich zu Volumenteilen wie kg zu 1.

## Beispiel 1

Eine Suspension von 4,2 Gew.-Teilen Natriumdisulfid in 50 Vol.-Teilen Ethanol werden bei Raumtemperatur zu einer Lösung von 35,2 Gew.-Teilen 3-(2'-Chlor-4'-trifluormethylphenoxy)-6-nitro-chlorbenzol in 100 Vol.-Teilen Ethanol zugefügt, und die Reaktionsmischung wird 40 Stunden nachgerührt. Anschließend wird der Niederschlag abgesaugt, mit Wasser verrührt und erneut abgesaugt. Man erhält 31 Gew.-Teile Bis-[3-(2'-chlor-4'-trifluormethylphenoxy)-6-nitrophenyl]-disulfid (= 89% Ausbeute) vom Fp. 161—165°C (Verbindung Nr. 1).

## Beispiel 2

Eine Suspension von 34,9 Gew.-Teilen 3-(2'-Chlor-4'-trifluormethylphenoxy)-6-nitrophenyldisulfid in 200 Vol.-Teilen absolutem Methylenchlorid werden bei Raumtemperatur mit Chlor gesättigt und 12 Stunden nachgerührt. Die Reaktionslösung wird abfiltriert, das Filtrat unter vermindertem Druck eingeengt und der ölige Rückstand durch Anreiben kristallisiert. Es werden 37 Gew.-Teile 3-(2'-Chlor-4'-trifluormethylphenoxy)-6-nitro-benzen-sulfenylchlorid (= 96% Ausbeute) vom Fp. 74—78°C (Verbindung Nr. 2) erhalten.

## Beispiel 3

Eine Lösung von 19,2 Gew.-Teilen 3-(2'-Chlor-4'-trifluormethylphenoxy)-6-nitro-benzen-sulfenylchlorid in 100 Vol.-Teilen absolutem Ether werden bei Raumtemperatur tropfenweise mit 5,3 Gew.-Teilen Thioglykolsäuremethylester versetzt und die Reaktionsmischung wird zwei Stunden nachgerührt. Anschließend wird unter vermindertem Druck zur Trockene eingeengt und der ölige Rückstand mit

Diisopropylether kristallisiert. Man erhält 25 Gew.-Teile 3-(2'-Chlor-4'-trifluormethylphenoxy)-6-nitrophenyl-methoxycarbonyl-methyl-disulfid (= 90% Ausbeute) vom Fp. 66—68° C (Verbindung Nr. 3).

Beispiel 4

Eine Lösung von 10 Gew.-Teilen 3-(2'-Chlor-4'-trifluormethylphenoxy)-6-nitro-benzen-sulfenylchlorid in 100 Vol.-Teilen absolutem Ether wird gleichzeitig mit 1,62 Gew.-Teilen Ethanthiol und 2,6 Gew.-Teilen Triethylamin versetzt und 2 Stunden bei Raumtemperatur nachgerührt. Die Reaktionsmischung wird filtriert, das Filtrat mit verdünnter wäßriger Salzsäure und Wasser extrahiert, über Magnesiumsulfat getrocknet, filtriert und unter vermindertem Druck eingeengt. Man erhält 8 Gew.-Teile 3-(2'-Chlor-4'-trifluormethyl-phenoxy)-6-nitrophenyl-ethyl-disulfid (= 98% Ausbeute) mit dem Brechungsindex $n_D^{25}$ : 1,6067 (Verbindung Nr. 4).

Beispiel 5

Zu einer Lösung von 19,2 Gew.-Teilen 3-(2'-Chlor-4'-trifluormethylphenoxy)-6-nitro-benzen-sulfenylchlorid in 100 Vol.-Teilen Ether werden 3,1 g Methylamin in Form einer 40%igen wäßrigen Lösung zugefügt. Es wird 2 Stunden bei Raumtemperatur nachgerührt, die organische Phase wird abgetrennt, mit Magnesiumsulfat getrocknet, filtriert und unter vermindertem Druck eingeengt. Man erhält 17,5 Gew.-Teile 3-(2'-Chlor-4'-trifluormethylphenoxy)-6-nitro-benzen-N-methyl-sulfenamid (= 95% Ausbeute) mit dem Brechungsindex $n_D^{25}$: 1,6109 (Verbindung Nr. 5).

Entsprechend können beispielsweise die folgenden Verbindungen der Formel

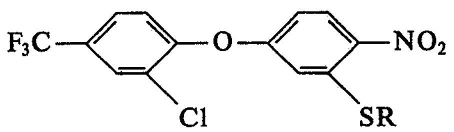

erhalten werden.

| Beispiel Nr. | R | Fp [°C]; $n_D^{25}$; | Wellenlänge einer Bande im IR-Spektrum |
|---|---|---|---|
| 6 | HS- | | |
| 7 | Methylthio | | |
| 8 | n-Propylthio | | |
| 9 | n-Butylthio | | 1,5981 |
| 10 | Isopropylthio | | |
| 11 | s-Butylthio | 55–59 | |
| 12 | 2-Methyl-butylthio | 44–48 | |
| 13 | tert.-Butylthio | 80–85 | |
| 14 | Dodecylthio | | 1,5601 |
| 15 | Methoxymethylthio | | |
| 16 | Methoxyethylthio | | |
| 17 | Methylthiomethylthio | | |
| 18 | Methylthioethylthio | | |
| 19 | Dodecylthioethylthio | | 1,5679 |
| 20 | Dimethylaminoethylthio | | |
| 21 | Chlorethylthio | | |
| 22 | Fluorethylthio | | |
| 23 | Benzylthio | | |
| 24 | 4-Chlorbenzylthio | 89–94 | |
| 25 | Phenethylthio | 68–72 | |
| 26 | Ethenylthio | | |
| 27 | Ethinylthio | | |
| 28 | $—S—CH_2COOH$ | | |
| 29 | $—S—CH(CH_3)—COOH$ | | |
| 30 | $—S—CH_2COOC_3H_7(n)$ | | |
| 31 | $—S—CH_2COOC_2H_5$ | 80–83 | |
| 32 | $—S—CH(CH_3)COOCH_3$ | | |
| 33 | $—S—CH(CH_3)COOC_2H_5$ | | |

Fortsetzung

| Beispiel Nr. | R | Fp [°C]; $n_D^{25}$; | Wellenlänge einer Bande im IR-Spektrum |
|---|---|---|---|
| 34 | $—S—CH_2CH_2COOC_{12}H_{25}$ | | |
| 35 | $—S—CH_2COOC_{18}H_{37}$ | | |
| 36 | $—S—CH_2CONHCH_3$ | | |
| 37 | $—S—CH(CH_3)CONHCH_3$ | | |
| 38 | Cyclopentylthio | | |
| 39 | Cyclohexylthio | 81–86 | |
| 40 | Phenylthio | | |
| 41 | 4-Chlorphenylthio | 81–84 | |
| 42 | 4-Methoxyphenylthio | | 1,6315 |
| 43 | 3-Chlorphenylthio | 81–85 | |
| 44 | 2,4-Dichlorphenylthio | | |
| 45 | 2,6-Dichlorphenylthio | | |
| 46 | 3-Trifluormethylphenylthio | 83–87 | |
| 47 | 4-Methylphenylthio | | |
| 48 | 2,4,5-Trichlorphenylthio | | |
| 49 | 2,5-Dichlorphenylthio | | |
| 50 | Pentachlorphenylthio | | |
| 51 | 2-Methylphenylthio | | |
| 52 | 2,3,4-Trichlorphenylthio | | |
| 53 | 4-Fluorphenylthio | | |
| 54 | Hydroxy | | |
| 55 | Methoxy | 59–62 | |
| 56 | Ethoxy | 64–68 | |
| 57 | n-Propoxy | 54–56 | |
| 58 | i-Propoxy | 65–68 | |
| 59 | n-Heptoxy | | 1,5669 |
| 60 | t-Butoxy | 98–102 | |

Fortsetzung

| Beispiel Nr. | R | Fp [°C]; $n_D^{25}$; | Wellenlänge einer Bande im IR-Spektrum |
|---|---|---|---|
| 61 | 2-Methoxyethoxy | 79–82 | |
| 62 | 2-Ethoxy-ethoxy | | |
| 63 | 2-Ethylthio-ethoxy | | |
| 64 | 2-Methylthio-ethoxy | | |
| 65 | 2-Chlorethoxy | 78–81 | |
| 66 | 2-Fluorethoxy | | |
| 67 | Benzyloxy | 101–104 | |
| 68 | 4-Chlor-benzyloxy | 109–112 | |
| 69 | 2-Phenyl-ethoxy | 72–75 | |
| 70 | Allyloxy | | |
| 71 | Propargyloxy | | |
| 72 | $—O—CH_2COOH$ | | |
| 73 | $—O—CH—COOH$<br>\|<br>$CH_3$ | | |
| 74 | $—O—CH_2COOCH_3$ | | |
| 75 | $—O—CH_2COOC_2H_5$ | | |
| 76 | $—O—CHCOOCH_3$<br>\|<br>$CH_3$ | | |
| 77 | $—O—CHCOOC_2H_5$<br>\|<br>$CH_3$ | 70–74 | |
| 78 | $—O—CH_2COOC_{12}H_{25}$ | | |
| 79 | $—O—CH_2CONHCH_3$ | | |
| 80 | $—O—CHCONHCH_3$<br>\|<br>$CH_3$ | | |
| 81 | Cyclopentoxy | 83–87 | |
| 82 | Cyclohexoxy | 79–83 | |
| 83 | Phenoxy | | |

Fortsetzung

| Beispiel Nr. | R | Fp [°C]; $n_D^{25}$; | Wellenlänge einer Bande im IR-Spektrum |
|---|---|---|---|
| 84 | 4-Chlorphenoxy | | 1,6148 |
| 85 | 3-Chlorphenoxy | | |
| 86 | 4-Fluor-phenoxy | | |
| 87 | 2,4-Dichlorphenoxy | | 1,6194 |
| 88 | 4-Methoxyphenoxy | | |
| 89 | 3-Trifluormethylphenoxy | 77–80 | |
| 90 | 4-Methylphenoxy | | |
| 91 | 2,6-Dichlorphenoxy | | |
| 92 | 2,4-Dibromphenoxy | | |
| 93 | 3-Bromphenoxy | | |
| 94 | 3-Fluorphenoxy | | |
| 95 | 3,5-Dimethylphenoxy | | 1,6141 |
| 96 | Ethylamino | | |
| 97 | n-Propylamino | | |
| 98 | Isopropylamino | | 1,5871 |
| 99 | n-Butylamino | | 1,5832 |
| 100 | Isobutylamino | 58–63 | |
| 101 | t-Butylamino | 101–104 | |
| 102 | n-Hexylamino | | 1,5736 |
| 103 | n-Dodecylamino | | 1,5494 |
| 104 | 2-Ethyl-butyl-amino | | 1,5736 |
| 105 | Pent-2-ylamino | | |
| 106 | 1,1-Dimethyl-butyl-amino | 66–69 | |
| 107 | Pent-3-yl-amino | | |
| 108 | Heptadecylamino | | |
| 109 | n-Heptylamino | | 1,5772 |
| 110 | 2,2-Dimethylpropylamino | 98–102 | |

Fortsetzung

| Beispiel Nr. | R | Fp [°C]; $n_D^{25}$; | Wellenlänge einer Bande im IR-Spektrum |
|---|---|---|---|
| 111 | Tetradecylamino | 109–112 | |
| 112 | (5-Methyl)-2-octyl-amino | | |
| 113 | (2,4-Dimethyl)-3-pentyl-amino | | |
| 114 | (5-Methyl)-3-heptyl-amino | | |
| 115 | (2,6-Dimethyl)-4-heptyl-amino | | |
| 116 | tert.-Octyl-amino | | |
| 117 | Cetylamino | 45–48 | |
| 118 | 2-Hydroxyethylamino | | 1,5965 |
| 119 | 2-Methoxyethylamino | | 3380 $cm^{-1}$ (NH) |
| 120 | 3-Methoxypropylamino | | |
| 121 | 3-Isopropoxy-propylamino | | |
| 122 | Di-methoxyethyl-amino | | 1,5711 |
| 123 | 3-n-Hexyloxypropylamino | | |
| 124 | 3-Lauryloxypropylamino | | |
| 125 | Ethoxy-sec.butyl-amino | | |
| 126 | 2-Benzylthioethylamino | | |
| 127 | 2-Octylthioethylamino | | |
| 128 | 4-Ethylthio-tert.butylamino | | |
| 129 | Methylthioisopropylamino | | |
| 130 | Methylthiopropylamino | | |
| 131 | Methylthioethylamino | | 1,6087 |
| 132 | Methylaminoethylamino | | |
| 133 | Ethylaminoethylamino | | |
| 134 | Dimethylaminoethylamino | | |
| 135 | Diethylaminoethylamino | | |
| 136 | Chlorethylamino | | |
| 137 | Fluorethylamino | | |

Fortsetzung

| Beispiel Nr. | R | Fp [°C]; $n_D^{25}$; | Wellenlänge einer Bande im IR-Spektrum |
|---|---|---|---|
| 138 | Benzylamino | | |
| 139 | Phenethylamino | | 1,6121 |
| 140 | Allylamino | | |
| 141 | Propargylamino | | |
| 142 | $-NH-CH_2-COOH$ | | |
| 143 | $-NH-CHCOOH$<br>│<br>$CH_3$ | | |
| 144 | $-NH-CH_2COOCH_3$ | | |
| 145 | $-NH-CH(CH_3)COOCH_3$ | | |
| 146 | $-NH-CH_2COOC_2H_5$ | | |
| 147 | $-NH-CH(CH_3)COOC_2H_5$ | | |
| 148 | $-NH-CH_2CONHCH_3$ | | |
| 149 | Cyclopropylamino | | |
| 150 | Cyclobutylamino | | |
| 151 | Cyclopentylamino | | |
| 152 | Cyclohexylamino | | |
| 153 | Phenylamino | 105–110 | |
| 154 | 4-Chlorphenyl-amino | 95–98 | |
| 155 | 3-Trifluormethylphenylamino | 90–95 | |
| 156 | 4-Fluorphenylamino | | |
| 157 | 4-Methylphenylamino | | |
| 158 | 4-Cyanphenylamino | | |
| 159 | 4-Nitrophenylamino | | |
| 160 | 3,4-Dichlorphenylamino | 55–60 | |
| 161 | 4-Methoxyphenylamino | | |
| 162 | 3,5-Ditrifluormethylphenylamino | | |
| 163 | 3-Methyl-4-fluorphenylamino | | |

Fortsetzung

| Beispiel Nr. | R | Fp [°C]; $n_D^{25}$; | Wellenlänge einer Bande im IR-Spektrum |
|---|---|---|---|
| 164 | 2,6-Difluorphenylamino | | |
| 165 | 3,4,5-Trifluorphenylamino | | |
| 166 | 3-Trifluormethoxyphenylamino | | |
| 167 | 3-Tetrafluorethoxyphenylamino | | |
| 168 | —NH—OH | | |
| 169 | Methoxyamino | | |
| 170 | Ethoxyamino | | |
| 171 | Benzyloxyamino | | |
| 172 | Phenoxyamino | | |
| 173 | 4-Chlorphenoxyamino | | |
| 174 | 4-Methylphenoxyamino | | |
| 175 | $—N(CH_3)(OCH_3)$ | 58–61 | |
| 176 | $—N(CH_3)(OC_2H_5)$ | | |
| 177 | $—N(OCH_3)$(Cyclohexyl) | | |
| 178 | $—NH—NH_2$ | | |
| 179 | $—NH—NH(CH_3)$ | | |
| 180 | $—NH—N(CH_3)_2$ | | |
| 181 | $—NH—NH(C_6H_5)$ | | |
| 182 | —NH—NH (4-Chlorphenyl) | | |
| 183 | $—N(CH_3)—NH(C_6H_5)$ | | |
| 184 | $—N(CH_3)—N(CH_3)C_6H_5$ | | |
| 185 | Morpholino | 126–130 | |
| 186 | 2,6-Dimethylmorpholino | | |
| 187 | Piperazino | | |
| 188 | 2,6-Dimethylpiperazino | | |
| 189 | N-Methyl-piperazino | | |
| 190 | Amino | 82–86 | |

Fortsetzung

| Beispiel Nr. | R | Fp [°C]; $n_D^{25}$; | Wellenlänge einer Bande im IR-Spektrum |
|---|---|---|---|
| 191 | Dimethylamino | | 1,6022 |
| 192 | 2-Methyl-n-butylamino | 47–49 | |
| 193 | 1-Ethyl-n-propylamino | 70–73 | |
| 194 | 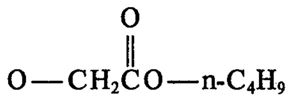 | | 1,5504 |
| 195 | 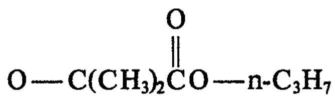 | | 1,5181 |
| 196 | 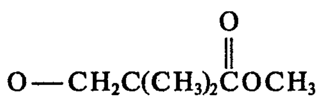 | | 1,5710 |
| 197 | 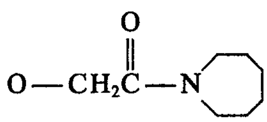 | | 1,5653 |
| 198 | 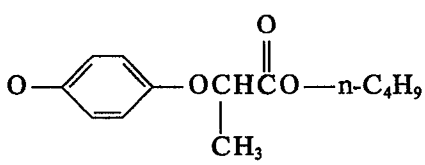 | | 1,5430 |

Beispielsweise können auch folgende andere Diphenylether der Formel I erhalten werden:

| Beispiel Nr. | 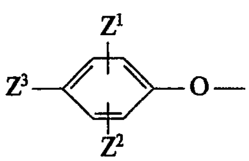 | R | Fp [°C]; $n_D^{25}$; Wellenlänge einer Bande im IR-Spektrum |
|---|---|---|---|
| 199 | 2,4-Dichlorphenoxy | Ethylthio | |
| 200 | desgl. | Methylamino | |
| 201 | desgl. | Phenylamino | |
| 202 | 2,4-Dibromphenoxy | Ethylthio | |
| 203 | desgl. | Methylamino | |
| 204 | 3-Chlor-4-trifluormethylphenoxy | Ethylthio | |
| 205 | desgl. | Methylamino | |
| 206 | 2,6-Dichlor-4-trifluormethylphenoxy | Ethylthio | |

Fortsetzung

| Beispiel Nr. | 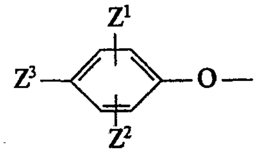 | R | Fp [°C]; $n_D^{25}$; Wellenlänge einer Bande im IR-Spektrum |
|---|---|---|---|
| 207 | 2,6-Dichlor-4-trifluormethylphenoxy | $-S-CH_2COOCH_3$ | |
| 208 | desgl. | $-S-CH(CH_3)COOCH_3$ | |
| 209 | desgl. | Methylamino | |
| 210 | desgl. | Benzylamino | |
| 211 | desgl. | Methoxyamino | |
| 212 | desgl. | Benzyloxyamino | |
| 213 | desgl. | $-N(CH_3)(OCH_3)$ | |

Die Diphenylether der Formel I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwekken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Steu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulte, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:

I. Man vermischt 90 Gewichtsteile des Wirkstoffs Nr. 3 mit 10 Gewichtsteilen N-Methyl-$\alpha$-pyrrolidon und erhält eine Lösung, die zur Anwendung in Form kleinster Tropfen geeignet ist.

II. 10 Gewichtsteile des Wirkstoffs Nr. 4 werden in einer Mischung gelöst, die aus 90 Gewichtsteilen Xylol, 6 Gewichtsteilen des Anlagerungsproduktes von 8 bis 10 Mol Ethylenoxid an 1 Mol Ölsäure-N-mono-ethanolamid, 2 Gewichtsteilen Calciumsalz der Dodecyl-benzol-sulfonsäure und 2 Gewichtsteilen des Anlagerungsproduktes von 40 Mol Ethylenoxid an 1 Mol Ricinusöl besteht.

III. 20 Gewichtsteile des Wirkstoffs Nr. 31 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Ricinusöl besteht.

IV. 20 Gewichtsteile des Wirkstoffs Nr. 2 werden in einer Mischung gelöst, die aus 25 Gewichtsteilen Cyclohexanol, 65 Gewichtsteilen einer Mineralöl-fraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 80 Gewichtsteile des Wirkstoffs Nr. 41 werden mit 3 Gewichtsteilen des Natriumsalzes der Diisobutyl-naphthalin-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.

VI. 5 Gewichtsteile des Wirkstoffs Nr. 55 werden mit 95 Gewichtsteilen feinteiligem Kaolin vermischt. Man erhält auf diese Weise ein Stäubemittel, das 5 Gewichtsprozent des Wirkstoffs enthält.

VII. 30 Gewichtsteile des Wirkstoffs Nr. 56 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile des Wirkstoffs Nr. 131 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation kann im Vorauflauf- oder im Nachauflaufverfahren erfolgen. Vorzugsweise werden die neuen Wirkstoffe bzw. diese enthaltende Mittel nach dem Auflaufen der unerwünschten Pflanzen ausgebracht. Sind die Wirkstoffe für gewisse Kulturpflanzen weniger verträglich, können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,005 bis 3 kg/ha.

Die Wirkung der Diphenylether der Formel I auf das Wachstum von erwünschten und unerwünschten Pflanzen läßt sich durch Gewächshausversuche zeigen:

Als Kulturgefäße dienen Plastikblumentöpfe mit 300 $cm^3$ Inhalt und lehmigen Sand mit etwa 1,5% Humus als Substrat. Die Samen der Testpflanzen wurden nach Arten getrennt flach eingesät.

Unmittelbar danach erfolgt bei Vorauflaufbehandlung das Aufbringen der Wirkstoffe bzw. der sie enthaltenden herbiziden Mittel auf die Erdoberfläche. Sie werden hierzu in Wasser als Verteilungsmittel suspendiert oder emulgiert und mittels fein verteilender Düsen gespritzt. Bei dieser Applikationsmethode beträgt die Aufwandmenge 3,0 kg Wirkstoff/ha. Nach dem Aufbringen der Mittel beregnet man die Gefäße leicht, um Keimung und Wachstum in Gang zubringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelt sie danach. Die für die Nachauflaufanwendung benutzten Reis- und Sojapflanzen werden in einem mit Torfmull (peat) angereicherten Substrat angezogen. Eine Beeinträchtigung der Ergebnisse ist nicht zu befürchten, da die Pflanzen erst nach dem Auflaufen mit Herbiziden behandelt werden. Zur Nachauflaufbehandlung werden entweder direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Aufwandmengen variieren je nach Wirkstoff zwischen 0,03 und 0,25 kg/ha.

Die Versuchsgefäße werden im Gewächshaus aufgestellt, wobei für wärmeliebende Arten wärmere Bereiche (20 bis 35° C) und für solche gemäßigter Klimate 10 bis 25° C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen:

Amaranthus spp. (Fuchsschwanz), Arachys hypogaea (Erdnüsse), Chenopodium album (weißer Gänsefuß), Euphorbia geniculata (Südamerik. Wolfsmilchart), Glycine max (Soja), Ipomoea spp. (Prunkwindearten), Lamium spp. (Taubnessel), Oryza sativa (Reis), Sida spinosa, Sinapis alba (Weißer Senf), Solanum nigrum (Schwarzer Nachtschatten), Triticum aestivum (Weizen), Viola tricolor (Stiefmütterchen).

Bei der Prüfung auf selektive herbizide Eigenschaften bekämpfen die Wirkstoffe Nr. 2, 3, 4, 5 und 31 breitblättige Unkräuter unter selektiver Schonung von Kulturpflanzen. Ebenso zeigen in diesen Prüfungen die Verbindungen Nr. 41, 42, 43, 98 und 131 eine sehr gute herbizide Wirkung, wobei Kulturpflanzen, wie Erdnüsse und Reis, nicht oder nur gering und vorübergehend geschädigt werden. Die Wirkstoffe Nr. 55 und 77 bzw. 82, 87 und 106 zeigen bei einer Aufwandmenge von 0,06 bzw. 0,25 kg Wirkstoff/ha eine gute Wirkung gegen breitblättrige Unkräuter ohne — oder mit nur temporären und geringen — Schädigungen an Weizen. Außerdem bekämpft beispielsweise die Verbindung Nr. 56 unerwünschte breitblättrige Pflanzen mit 0,06 kg/ha sehr gut, wobei sie sich in verschiedenen Kulturen selektiv verhält.

Eine herbizide Wirkung bei Vorauflaufanwendung zeigen beispielsweise die Verbindungen Nr. 56, 194, 197 und 198 bei einer Aufwandmenge von 3,0 kg Wirkstoff/ha.

In Anbetracht der Vielseitigkeit der Applikationsmethoden können die erfindungsgemäßen Verbindungen noch in einer weiteren Zahl von Kulturpflanzen zur Beseitigung von unerwünschtem Pflanzenwuchs, vorzugsweise breitblättriger annueller Arten, eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napobrassica | Kohlrübe |
| Camellia sinensis | Teestrauch |
| Carthamus tinctorius | Saflor — Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |

(Fortsetzung)

| Botanischer Name | Deutscher Name |
|---|---|
| Coffea arabica (Coffea canephora, Coffea liberica) | Kaffee |
| Cucumis melo | Melone |
| Cynodon dactylon | Bermudagras |
| Elaeis guineensis | Ölpalme |
| Fragaria vesca | Erdbeere |
| Glycine max | Sojabohne |
| Gossypium hirsutum (Gossypium arboreum Gossypium herbaceum Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Metha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |

(Fortsetzung)

| Botanischer Name | Deutscher Name |
|---|---|
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Solanum tuberosum | Kartoffel |
| Sorghum bicolor (s. vulgare) | Mohrenhirse |
| Sorghum dochna | Zuckerhirse |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die erfindungsgemäßen Verbindungen auch mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Triazinone, Uracile, Benzofuranderivate, Cyclohexan-1,3-dion-derivate und andere in Betracht.

Außerdem kann es von Nutzen sein, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Bekämpfung von Ernährungs- und Spurenelementmängeln eingesetzt werden. Es können auch nichtphytotoxische Öle und Ölkonzentrate zugesetzt werden.

**Patentansprüche**

1. Diphenylether der Formel

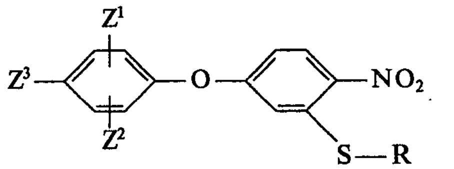

(I)

in der

$Z^1$ und $Z^2$ jeweils unabhängig voneinander Wasserstoff, Halogen, Nitro, Cyano, Carboxy, $C_1-C_4$-Alkyl, $C_1-C_4$-Halogenalkyl oder $C_1-C_4$-Alkoxy,

$Z^3$ Halogen, Nitro, Cyano, $C_1-C_4$-Alkyl, $C_1-C_4$-Halogenalkyl, $C_1-C_4$-Alkoxy, $C_1-C_4$-Halogenalkoxy, $C_1-C_4$-Alkylmercapto, $C_1-C_4$-Halogenalkylmercapto, $C_1-C_4$-Alkylsulfinyl, $C_1-C_4$-Halogenalkylsulfinyl, $C_1-C_4$-Alkylsulfonyl oder $C_1-C_4$-Halogenalkylsulfonyl und

R Wasserstoff, Halogen oder den Rest $-X-R^1$, wobei X für Sauerstoff und Schwefel und $R^1$ für Wasserstoff, $C_1-C_{20}$-Alkyl, $C_1-C_{20}$-Alkoxyalkyl, $C_1-C_{20}$-Alkylthioalkyl, $C_2-C_{20}$-Alkylaminoalkyl, $C_3-C_{20}$-Dialkylaminoalkyl, $C_1-C_{20}$-Halogenalkyl, einen gegebenenfalls durch Halogen substituierten araliphatischen Rest mit 7 bis 20 Kohlenstoffatomen, $C_2-C_8$-Alkenyl, $C_2-C_8$-Halogenalkenyl oder $C_2-C_8$-Alkinyl mit bis zu 8 Kohlenstoffatomen, Carboxyalkyl, Alkoxycarbonylalkyl oder Carbamoylalkyl mit bis zu 6 Kohlenstoffatomen, Cycloalkyl mit 3 bis 20 Kohlenstoffatomen oder gegebenenfalls substituiertes Phenyl stehen,
oder den Rest $-NR^2R^3$, wobei $R^2$ und $R^3$ gleich oder verschieden sind und die für $R^1$ genannten Bedeutungen haben oder wobei für den Fall, daß $R^3$ für Wasserstoff oder $C_1-C_4$-Alkyl steht, $R^2$ für Hydroxy, $C_1-C_{20}$-Alkoxy, durch $C_1-C_6$-Alkoxy, $C_1-C_6$-Alkylthio, $C_1-C_6$-Alkylamino, $C_2-C_6$-Dialkylamino, durch Halogen, Carboxyalkyl, Alkoxycarbonyl oder Phenyl substituiertes $C_1-C_{20}$-Alkoxy, gegebenenfalls substituiertes Phenoxy, Amino, durch $C_1-C_{20}$-Alkyl oder Phenyl substituiertes Amino steht oder wobei $R^2$ und $R^3$ zusammen für eine gegebenenfalls durch $C_1-C_4$-Alkyl ein- oder mehrfach substituierte Alkylenkette mit 4 oder 5 Kohlenstoffatomen stehen, die durch Sauerstoff oder Stickstoff unterbrochen sein kann, bedeuten.

2. Diphenylether der Formel I gemäß Anspruch 1, dadurch gekennzeichnet, daß $Z^1$, $Z^2$ und $Z^3$ in 2,4,6-Stellung stehen und unabhängig voneinander Halogen, $C_1-C_4$-Halogenalkyl oder $C_1-C_4$-Halogenalkylmercapto und R Halogen, den Rest $-X-R^1$, wobei X für Sauerstoff und Schwefel und $R^1$ für $C_1-C_8$-Alkyl, $C_2-C_6$-Alkoxyalkyl, $C_2-C_6$-Carboxyalkyl, $C_3-C_6$-Alkoxycarbonylalkyl stehen, oder den Rest $-NR^2R^3$, wobei $R^2$ und $R^3$ gleich oder verschieden sind und für Wasserstoff, $C_1-C_{10}$-Alkyl, $C_2-C_6$-Alkoxyalkyl oder $C_2-C_6$-Alkylthioalkyl oder gegebenenfalls durch Halogen substituiertes Phenyl oder wobei $R^3$ für Wasserstoff oder $C_1-C_4$-Alkyl und $R^2$ für $C_1-C_4$-Alkoxy, Amino oder durch $C_1-C_8$-Alkyl substituiertes Amino stehen.

3. Diphenylether der Formel I gemäß Anspruch 1, dadurch gekennzeichnet, daß $Z^1$ Wasserstoff, $Z^2$ und $Z^3$ Halogen oder $C_1-C_4$-Halogenalkyl und R den Rest $-X-R^1$, wobei X für Sauerstoff und $R^1$ für $C_1-C_4$-Alkyl stehen, bedeuten.

4. 3-(2'-Chlor-4'-trifluormethyl-phenoxy)-6-nitro-benzen-N-methyl-sulfen-amid.

5. 3-(2'-Chlor-4'-trifluormethyl-phenoxy)-6-nitro-benzen-sulfensäuremethylester.

6. Verfahren zur Herstellung von Diphenylethern der Formel I gemäß Anspruch 1, in der R Halogen

bedeutet und $Z^1$, $Z^2$ und $Z^3$ die in Anspruch 1 genannten Bedeutungen haben, dadurch gekennzeichnet, daß man einen Ether der Formel

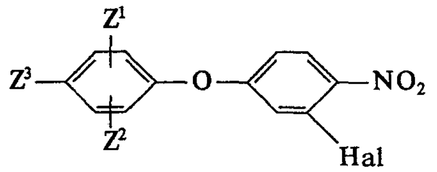

(II)

in der $Z^1$, $Z^2$ und $Z^3$ die obengenannten Bedeutungen haben und Hal für Halogen steht, mit der mindestens stöchiometrischen Menge Natriumdisulfid in Gegenwart eines organischen Lösungsmittels bei Temperaturen im Bereich von 0 bis 120° C zu dem Disulfid der Formel

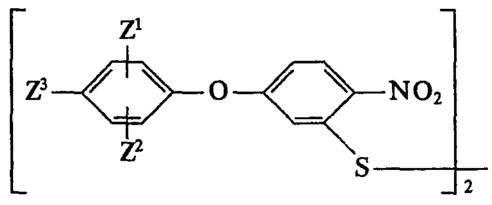

(III)

in der $Z^1$, $Z^2$ und $Z^3$ die im Anspruch 1 genannten Bedeutungen haben, und dieses anschließend mit der mindestens stöchiometrischen Menge an elementarem Halogen in Gegenwart eines inerten organischen Lösungsmittels bei einer Temperatur im Bereich von −20 bis +120° C umsetzt.

7. Verfahren zur Herstellung von Diphenylethern der Formel I gemäß Anspruch 1, in der R Wasserstoff, den Rest $-XR^1$ oder den Rest $-NR^2R^3$ bedeutet und $Z^1$, $Z^2$ und $Z^3$ die im Anspruch 1 genannten Bedeutungen haben, dadurch gekennzeichnet, daß man ein Sulfenylchlorid der Formel

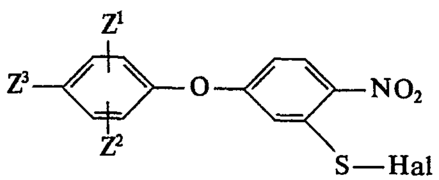

(IV)

in der $Z^1$, $Z^2$ und $Z^3$ die im Anspruch 1 genannten Bedeutungen haben und Hal für Halogen steht, mit der mindestens äquimolaren Menge einer Verbindung der Formel

$R-H$ (V)

in der R die obengenannten Bedeutungen hat, umsetzt.

8. Herbizid, enthaltend einen Diphenylether der Formel I gemäß Anspruch 1.

9. Herbizid, enthaltend inerte Zusatzstoffe und einen Diphenylether der Formel I gemäß Anspruch 1.

10. Verfahren zur Bekämpfung unerwünschten Pflanzenwachstums, dadurch gekennzeichnet, daß man die unerwünschten Pflanzen oder die von unerwünschtem Pflanzenwachstum freizuhaltende Fläche mit einer herbizid wirksamen Menge eines Diphenylethers der Formel I gemäß Anspruch 1 behandelt.

**Claims**

1. A diphenyl ether of the formula

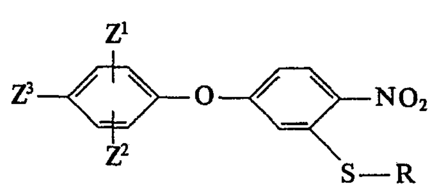

(I)

where $Z^1$ and $Z^2$ independently of one another are hydrogen, halogen, nitro, cyano, carboxyl, $C_1-C_4$-alkyl, $C_1-C_4$-haloalkyl or $C_1-C_4$-alkoxy, $Z^3$ is halogen, nitro, cyano, $C_1-C_4$-alkyl, $C_1-C_4$-haloal-

kyl, $C_1-C_4$-alkoxy, $C_1-C_4$-haloalkoxy, $C_1-C_4$-alkylmercapto, $C_1-C_4$-haloalkylmercapto, $C_1-C_4$-alkylsulfinyl, $C_1-C_4$-haloalkylsulfinyl, $C_1-C_4$-alkylsulfonyl or $C_1-C_4$-haloalkylsulfonyl, and R is hydrogen, halogen or $-X-R^1$, where X is oxygen or sulfur and $R^1$ is hydrogen, $C_1-C_{20}$-alkyl, $C_1-C_{20}$-alkoxyalkyl, $C_1-C_{20}$-alkylthioalkyl, $C_2-C_{20}$-alkylaminoalkyl, $C_3-C_{20}$-dialkylaminoalkyl, $C_1-C_{20}$-haloalkyl, an unsubstituted or halogen-substituted araliphatic radical of 7 to 20 carbon toms, $C_2-C_8$-alkenyl, $C_2-C_8$-haloalkenyl or $C_2-C_8$-alkynyl of up to 8 carbon atoms, carboxyalkyl, alkoxycarbonylalkyl or carbamylalkyl of up to 6 carbon atoms, cycloalkyl of 3 to 20 carbon atoms or unsubstituted or substituted phenyl, or R is $-NR^2R^3$, where $R^2$ and $R^3$ are identical or different and have the meanings stated for $R^1$, or if $R^3$ is hydrogen or $C_1-C_4$-alkyl, $R^2$ is hydroxyl or $C_1-C_{20}$-alkoxy, or is $C_1-C_{20}$-alkoxy which is substituted by $C_1-C_6$-alkoxy, $C_1-C_6$-alkylthio, $C_1-C_6$-alkylamino, $C_2-C_6$-dialkylamino, halogen, carboxyalkyl, alkoxycarbonyl or phenyl, or is unsubstituted or substituted phenoxy or unsubstituted, $C_1-C_{20}$-alkyl-substituted or phenyl-substituted amino, or $R^2$ and $R^3$ together form an alkylene chain of 4 or 5 carbon atoms which is unsubstituted or monosubstituted or polysubstituted by $C_1-C_4$-alkyl and can be interrupted by oxygen or nitrogen.

2. A diphenyl ether of the formula I as claimed in claim 1, where $Z^1$, $Z^2$ and $Z^3$ are in the 2-, 4- and 6-positions and independently of one another are halogen, $C_1-C_4$-haloalkyl or $C_1-C_4$-haloalkylmercapto, and R is halogen, $-X-R^1$, where X is oxygen or sulfur and $R^1$ is $C_1-C_8$-alkyl, $C_1-C_6$-alkoxyalkyl, $C_2-C_6$-carboxyalkyl or $C_3-C_6$-alkoxycarbonylalkyl, or R is $-NR^2R^3$, where $R^2$ and $R^3$ are identical or different and are each hydrogen, $C_1-C_{10}$-alkyl, $C_2-C_6$-alkoxyalkyl, $C_2-C_6$-alkylthioalkyl or unsubstituted or halogen-substituted phenyl, or where $R^3$ is hydrogen or $C_1-C_4$-alkyl and $R^2$ is $C_1-C_4$-alkoxy or unsubstituted or $C_1-C_8$-alkylsubstituted amino.

3. A diphenyl ether of the formula I as claimed in claim 1, where $Z^1$ is hydrogen, $Z^2$ and $Z^3$ are halogen or $C_1-C_4$-haloalkyl and R is $-X-R^1$, X denoting oxygen and $R^1$ being $C_1-C_4$-alkyl.

4. 3-(2'-Chloro-4'-trifluoromethylphenoxy)-6-nitrobenzene-N-methylsulfenamide.

5. Methyl 3-(2'-chloro-4'-trifluoromethylphenoxy)-6-nitrobenzenesulfenate.

6. A process for the preparation of a diphenyl ether of the formula I as claimed in claim 1, where R is halogen and $Z^1$, $Z^2$ and $Z^3$ have the meanings given in claim 1, wherein an ether of the formula

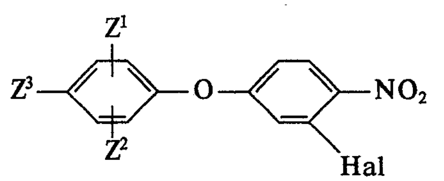

(II)

where $Z^1$, $Z^2$ and $Z^3$ have the above meanings and Hal is halogen, is reacted with not less than the stoichiometric amount of sodium disulfide, in the presence of an organic solvent at from 0 to 120°C to give the disulfide of the formula

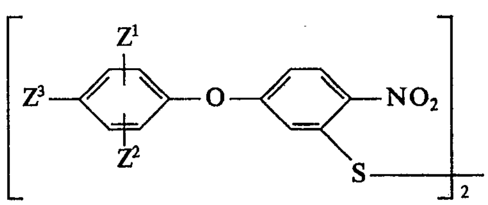

(III)

where $Z^1$, $Z^2$ and $Z^3$ have the meanings given in claim 1, and this disulfide is then reacted with not less than the stoichiometric amount of elementary halogen in the presence of an inert organic solvent at from −20 to +120°C.

7. A process for the preparation of a diphenyl ether of the formula I as claimed in claim 1, where R is hydrogen, $-XR^1$ or $-NR^2R^3$, and $Z^1$, $Z^2$ and $Z^3$ have the meanings given in claim 1, wherein a sulfenyl chloride of the formula

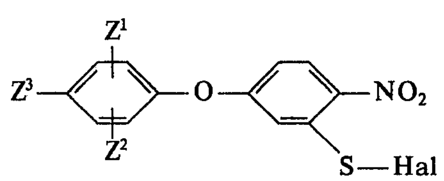

(IV)

where $Z^1$, $Z^2$ and $Z^3$ have the meanings given in claim 1, and Hal is halogen, is reacted with not less than an equimolar amount of a compound of the formula

$$R-H \qquad (V)$$

where R has the above meanings.

8. A herbicide containing a diphenyl ether of the formula I as claimed in claim 1.

9. A herbicide containing inert additives and a diphenyl ether of the formula I as claimed in claim 1.

10. A process for combating the growth of unwanted plants, wherein the unwanted plants or the area to be kept free from unwanted plant growth are treated with a herbicidally effective amount of a diphenyl ether of the formula I as claimed in claim 1.

**Revendications**

1. Diphényléthers de formule

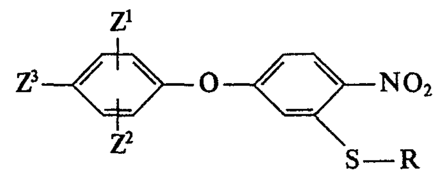

(I)

dans laquelle $Z^1$ et $Z^2$ représentent chacun, indépendamment l'un de l'autre, hydrogène, halogène, cyano, carboxy, alkyle en $C_1-C_4$, halogénoalkyle en $C_1-C_4$, ou alcoxy en $C_1-C_4$,
$Z^3$ halogène, nitro, cyano, alkyle en $C_1-C_4$, halogènoalkyle en $C_1-C_4$, alcoxy en $C_1-C_4$, halogènoalcoxy en $C_1-C_4$, alkylmercapto en $C_1-C_4$, halogénoalkylmercapto en $C_1-C_4$, alkylsulfinyle en $C_1-C_4$, halogénoalkylsulfinyle en $C_1-C_4$, alkylsulfonyle en $C_1-C_4$, ou halogénoalkylsulfonyle en $C_1-C_4$,
R hydrogène, halogène ou le reste $-X-R^1$,
X représentant oxygène et soufre et $R^1$ hydrogène, alkyle en $C_1-C_{20}$, alcoxyalkyle en $C_1-C_{20}$, alkylthioalkyle en $C_1-C_{20}$, alkylaminoalkyle en $C_2-C_{20}$, dialkylaminoalkyle en $C_3-C_{20}$, halogénoalkyle en $C_1-C_{20}$, un reste araliphatique à 7 à 20 atomes de carbone, éventuellement substitué par halogène, alcényle en $C_2-C_8$, halogénoalcényle en $C_2-C_8$, ou alcinyle en $C_2-C_8$, ayant jusqu'à 8 atomes de carbone, carboxyalkyle, alcoxycarbonylalkyle ou carbamoylalkyle ayant jusqu'à 6 atomes de carbone, cyclo-alkyle ayant 3 à 20 atomes de carbone ou phényle éventuellement substitué ou le reste $-NR^2R^3$, $R^2$ et $R^3$ étant identiques ou différents et ayant les significations indiquées pour $R^1$ ou, dans le cas où $R^3$ représente hydrogène ou alcényle en $C_1-C_4$, $R^2$ représentant hydroxy, alcoxy en $C_1-C_{20}$, alcoxy en $C_1-C_{20}$ substitué par alcoxy en $C_1-C_6$, alkylthio en $C_1-C_6$, alkylamino en $C_1-C_6$, dialkylamino en $C_2-C_6$, par halogène, carboxyalkyle, alcoxycarbonyl ou phényle, phénoxy éventuellement substitué, amino, amino substitué par alkyle en $C_1-C_{20}$ ou phényle, ou bien $R^2$ et $R^3$ représentant ensemble une chaîne à 4 ou 5 atomes de carbone éventuellement substituée une ou plusieurs fois par alkyle en $C_1-C_4$, qui peut être interrompue par oxygène ou azote.

2. Diphényléthers de formule I selon la revendication 1, caractérisés par le fait que $Z^1$, $Z^2$ et $Z^3$ se trouvent en position 2, 4, 6 et représentent, indépendamment l'un de l'autre, halogène, halogènoalkyle en $C_1-C_4$ ou halogénoalkylmercapto en $C_1-C_4$ et R, halogène, le reste $-X-R^1$, X représentant oxygène et soufre et $R^1$ étant mis pour alkyle en $C_1-C_8$, alcoxyalkyle en $C_2-C_6$, carboxyalkyle en $C_2-C_6$, alcoxycarbonylalkyle en $C_3-C_6$, ou le reste $-NR^2R^3$, $R^2$ et $R^3$ étant identiques ou différents et représentant hydrogène, alkyle en $C_1-C_{10}$, alcoxyalkyle en $C_2-C_6$ ou alkylthioalkyle en $C_2-C_6$ ou phényle éventuellement substitué par halogène, ou $R^3$ représentant hydrogène ou alkyle en $C_1-C_4$ et $R^2$ représentant alcoxy en $C_1-C_4$, amino ou amino substitué par alkyle en $C_1-C_8$.

3. Diphényléthers de formule I selon la revendication 1, caractérisés par le fait que $Z^1$ est mis pour hydrogène, $Z^2$ et $Z^3$ pour halogène ou halogénoalkyle en $C_1-C_4$ et R représente le reste $-X-R^1$, X étant mis pour oxygène et $R^1$ pour alkyle en $C_1-C_4$.

4. 3-(2'-chloro-4'-trifluorométhyl-phénoxy)-6-nitrobenzène-N-méthyl-sulfamide.

5. 3-(2'-chloro-4'-trifluorométhyl-phénoxy)-6-nitrobenzène-sulfonate de méthyle.

6. Procédé de préparation de diphényléthers de formule I selon la revendication 1, dans laquelle R représente halogène et $Z^1$, $Z^2$ et $Z^3$ ont les significations données dans la revendication 1, caractérisé par le fait que l'on fait réagir, en présence d'un solvant organique, à des températures de 0 à 120° C, un éther de formule

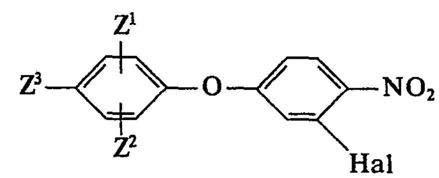

(II)

dans laquelle $Z^1$, $Z^2$ et $Z^3$ ont les significations sus-indiquées et Hal représente halogène, avec une quantité au moins stoéchiométrique de disulfure de sodium, pour obtenir le disulfure de formule

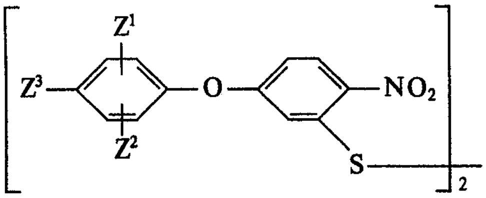

(III)

dans laquelle $Z^1$, $Z^2$ et $Z^3$ ont les significations indiquées dans la revendication 1, puis on fait réagir ce composé, en présence d'un solvant organique inerte, à une température de $-20$ à $+120°C$, avec la quantité au moins stoéchiométrique d'halogène élémentaire.

7. Procédé de préparation de diphényléthers de formule I selon la revendication 1 dans laquelle R représente hydrogène, le reste $-XR^1$ ou le reste $-NR^2R^3$ et $Z^1$, $Z^2$, $Z^3$ ont les significations données dans la revendication 1, caractérisé par le fait que l'on fait réagir un chlorure de sulfényle de formule

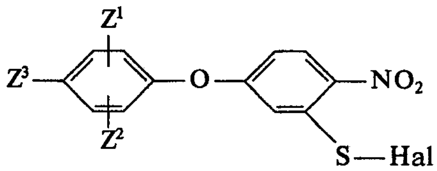

(IV)

dans laquelle $Z^1$, $Z^2$ et $Z^3$ ont les significations données dans la revendication 1 et Hal représente halogène, avec la quantité au moins moléculaire d'un composé de formule

R—H (V)

dans laquelle R a les significations sus-indiquées.

8. Herbicide contenant un diphényléther de formule I selon la revendication 1.

9. Herbicide contenant un additif inerte et un diphényléther de formule I selon la revendication 1.

10. Procédé de lutte contre la croissance de plantes indésirables, caractérisé par le fait que l'on traite les plantes indésirables, ou les surfaces à maintenir sans croissance indésirable de plantes, avec une quantité, active du point de vue herbicide, d'un diphényléther de formule I selon la revendication 1.